(19) Europäisches Patentamt
European Patent Office
Office européen des brevets

(11) **EP 4 621 076 A1**

(12) **EUROPEAN PATENT APPLICATION**
published in accordance with Art. 153(4) EPC

(43) Date of publication:
**24.09.2025 Bulletin 2025/39**

(21) Application number: **23907055.0**

(22) Date of filing: **19.12.2023**

(51) International Patent Classification (IPC):
$C21B\ 5/06^{(2006.01)}$    $C07C\ 1/04^{(2006.01)}$
$C07C\ 1/12^{(2006.01)}$    $C10K\ 3/06^{(2006.01)}$
$C21B\ 5/00^{(2006.01)}$    $C21B\ 13/02^{(2006.01)}$
$F27D\ 17/00^{(2025.01)}$

(52) Cooperative Patent Classification (CPC):
C07C 1/04; C07C 1/12; C10K 3/06; C21B 5/00;
C21B 5/06; C21B 13/02; F27D 17/00

(86) International application number:
**PCT/JP2023/045555**

(87) International publication number:
**WO 2024/135695 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2022 JP 2022207620**

(71) Applicant: **JFE Steel Corporation
Tokyo 100-0011 (JP)**

(72) Inventors:
• **MORIYA, Kota**
**Tokyo 100-0011 (JP)**
• **TAKAHASHI, Koichi**
**Tokyo 100-0011 (JP)**
• **TERUI, Koki**
**Tokyo 100-0011 (JP)**

(74) Representative: **Scott, Stephen John**
**YUJA IP LAW
4 Centenary House
The Avenue
York YO30 6AU (GB)**

(54) **REDUCED IRON PRODUCTION METHOD**

(57)    Provided is a method of producing reduced iron that can achieve both energy savings and a decrease in $CO_2$ emissions. A top gas circulation and reuse system is provided that includes a blowing process, a reduction process, a distribution process, a synthesis process, and a reforming process. An amount of gas introduced into the distribution process, the synthesis process, and the reforming process is controlled according to a $CO_2$ conversion rate from a methanation reaction in the synthesis process.

*FIG. 2*

EP 4 621 076 A1

**Description**

TECHNICAL FIELD

The present disclosure relates to a method of producing reduced iron.

BACKGROUND

In recent years, there has been a strong demand for energy savings in steelworks against the backdrop of global environmental issues and fossil fuel depletion issues. The raw material of iron is mainly iron oxide, and a reduction process to reduce this iron oxide is essential in steelworks. The most widespread and common reduction process worldwide uses a blast furnace. In a blast furnace, coke or pulverized coal reacts with oxygen in hot blast (air heated to about 1200 °C) from a tuyere. This reaction produces CO and $H_2$ as reducing gases, which are used to reduce the iron ore and the like in the furnace. Recent improvements in blast furnace operation technology have decreased the reducing agent rate (the amount of coke and pulverized coal used per tonne of hot metal produced) to about 500 kg/t, and the reducing agent rate has already almost reached a lower limit. Therefore, no further significant decrease in the reducing agent rate is expected.
On the other hand, in regions where natural gas is produced, a vertical reduction furnace (hereinafter also referred to as a shaft furnace) is often used to produce reduced iron. In this method, the reduction furnace is charged with sintered ore, pellets, or other agglomerated iron ore as the iron oxide raw material (hereinafter also simply referred to as iron oxide). Reducing gas including CO and $H_2$ is then blown into the reduction furnace to reduce the iron oxide to produce reduced iron. In this method, natural gas or the like is used as the feed gas for the reducing gas. This feed gas is heated and reformed together with top gas in a reformer. This produces reducing gas. Here, the top gas is gas after the iron oxide is reduced in the reduction furnace, and is typically discharged from the top of the reduction furnace. The reducing gas is blown into the reduction furnace and reacts with iron oxide supplied from the top of the furnace. The iron oxide is then reduced to reduced iron. The reduced iron is then cooled in a region below where the reducing gas is blown into the reduction furnace, and then discharged from the bottom of the reduction furnace.
Further, as mentioned above, the top gas, which is gas after iron oxide is reduced, is discharged from the reduction furnace, for example, from the top of the furnace. After dust collection and cooling is applied to the top gas, some is fed to the reformer as raw material for reformed gas. Further, remaining top gas is used as fuel gas for the reformer. In this method, the top gas used as fuel gas for the reformer is normally discharged out of the system.
As such a reduced iron production process, for example, Patent Literature (PTL) 1 describes a method of producing reduced iron by reforming exhaust gas from a reduction furnace and natural gas in a reformer to produce reducing gas consisting mainly of CO and $H_2$, blowing the reducing gas into the reduction furnace, and reducing iron oxide in the reduction furnace.
Further, PTL 2 describes a method of producing reduced iron by reforming coke oven gas and $CO_2$-removed top gas from a reduction furnace to produce reducing gas, which is then blown into a reduction furnace.

CITATION LIST

Patent Literature

PTL 1: JP 2017-088912 A
PTL 2: JP 6190522 B2

SUMMARY

(Technical Problem)

The method described in PTL 1 uses externally supplied natural gas for the production of reducing gas. Therefore, although lower than that of blast furnaces, there is a problem that a certain amount of $CO_2$ emissions are unavoidable.
Further, the method described in PTL 2 uses coke oven gas or converter gas produced in a steelworks to produce reducing gas. Here, in an integrated steelworks, coke oven gas and converter gas are needed as fuel gas for downstream processes such as a heating furnace and an annealing furnace. Therefore, when coke oven gas or converter gas is diverted to the reduced iron production process, a fuel gas shortage is caused in the downstream processes. As a result, natural gas is supplied from outside to compensate for the shortage of fuel gas in the downstream processes. That is, even with the method described in PTL 2, energy savings and a decrease in $CO_2$ emissions could not both be achieved, and the problem remained.

In view of the circumstances described, it would be helpful to provide a method of producing reduced iron that can realize both energy savings and a decrease in $CO_2$ emissions.

(Solution to Problem)

The inventors have conducted extensive research to realize both energy savings and a decrease in $CO_2$ emissions, and developed a system that circulates and reuses top gas.

That is, the inventors developed

a top gas circulation and reuse system (hereinafter also simply referred to a circulation system) comprising:

a blowing process of blowing reducing gas into a reduction furnace;
a reduction process, in the reduction furnace, of reducing iron oxide by the reducing gas to obtain reduced iron;
a distribution process of distributing top gas discharged from the reduction furnace into a first top gas and a second top gas;
a synthesis process of synthesizing regenerative methane gas from the first top gas and hydrogen gas; and
a reforming process of using the regenerative methane gas and the second top gas as feed gas to obtain the reducing gas from the feed gas.

The inventors further investigated and made the following discoveries.

- In the circulation system described above, an amount of gas introduced into the distribution process, the synthesis process, and the reforming process is controlled according to a $CO_2$ conversion rate $\eta_m$ [-] due to the methanation reaction in the synthesis process (hereinafter also referred to simply as the $CO_2$ conversion rate).
- This allows the production of reduced iron with high operational stability without the need for extra energy input, that is, production achieving both further energy savings and a decrease in $CO_2$ emissions.

The present disclosure is based on these discoveries and further studies.

Primary features of the present disclosure are as follows.

1. A method of producing reduced iron, the method comprising:

a charging process of charging iron oxide into a reduction furnace;
a blowing process of blowing reducing gas into the reduction furnace;
a reduction process, in the reduction furnace, of reducing the iron oxide by the reducing gas to obtain reduced iron;
a distribution process of distributing top gas discharged from the reduction furnace into a first top gas and a second top gas;
a synthesis process of synthesizing regenerative methane gas using the first top gas and hydrogen gas; and
a reforming process of using the regenerative methane gas and the second top gas as feed gas to obtain the reducing gas from the feed gas, wherein
an amount of gas introduced into the distribution process, the synthesis process, and the reforming process is controlled according to a $CO_2$ conversion rate $\eta_m$ from a methanation reaction in the synthesis process.

2. The method of producing reduced iron according to 1, above, wherein the amount of gas introduced into the distribution process, the synthesis process, and the reforming process is controlled so as to satisfy the following Expressions (1) and (2),

[Math 1]

$$1 - \frac{V_{CO2}}{W_{CO2}} \leq \eta_m$$

...(1)

[Math 2]

$$1 - \frac{V_{H2}}{W_{H2}+E_{H2}} \leq \eta_m \leq 1 + \frac{W_{H2}-V_{H2}}{E_{H2}}$$

...(2)

where

$W_{CO2}$ is an amount of $CO_2$, in $Nm^3/t$, contained in the top gas introduced into the distribution process,

$W_{H2}$ is an amount of $H_2$, in $Nm^3/t$, contained in the top gas introduced into the distribution process,

$E_{H2}$ is an amount of $H_2$, in $Nm^3/t$, contained in the hydrogen gas introduced into the synthesis process,

$V_{CO2}$ is an amount of $CO_2$, in $Nm^3/t$, contained in the gas introduced into the reforming process, and

$V_{H2}$ is an amount of $H_2$, in $Nm^3/t$, contained in the gas introduced into the reforming process.

3. The method of producing reduced iron according to 1 or 2, above, wherein, in the distribution process, the top gas is separated into separated carbon dioxide gas and first remaining gas,

the separated carbon dioxide gas is distributed as the first top gas, and
the first remaining gas is distributed as the second top gas.

4. The method of producing reduced iron according to 1 or 2, above, wherein, in the distribution process, the top gas is separated into separated carbon dioxide gas and first remaining gas,

the separated carbon dioxide gas is distributed as the first top gas, and
the first remaining gas is separated into separated hydrogen gas and second remaining gas,
the separated hydrogen gas is introduced into the synthesis process, and
the second remaining gas is distributed as the second top gas.

5. The method of producing reduced iron according to 1 or 2, above, wherein, in the distribution process, the top gas is separated into separated hydrogen gas and third remaining gas,

the separated hydrogen gas is introduced into the synthesis process,
the third remaining gas is separated into separated carbon dioxide gas and fourth remaining gas, and
the separated carbon dioxide gas is distributed as the first top gas and the fourth remaining gas is distributed as the second top gas.

(Advantageous Effect)

According to the present disclosure, it is possible to operate with high operational stability and achieve both further energy savings and a decrease in $CO_2$ emissions in the production of reduced iron.

BRIEF DESCRIPTION OF THE DRAWINGS

In the accompanying drawings:

FIG. 1 is a diagram illustrating a conventional reduced iron production process; and
FIG. 2 is a diagram illustrating an example of a reduced iron production process according to a method of producing reduced iron according to an embodiment of the present disclosure.

DETAILED DESCRIPTION

The following is a description of a method of producing reduced iron according to an embodiment of the present disclosure, with reference to the drawings.

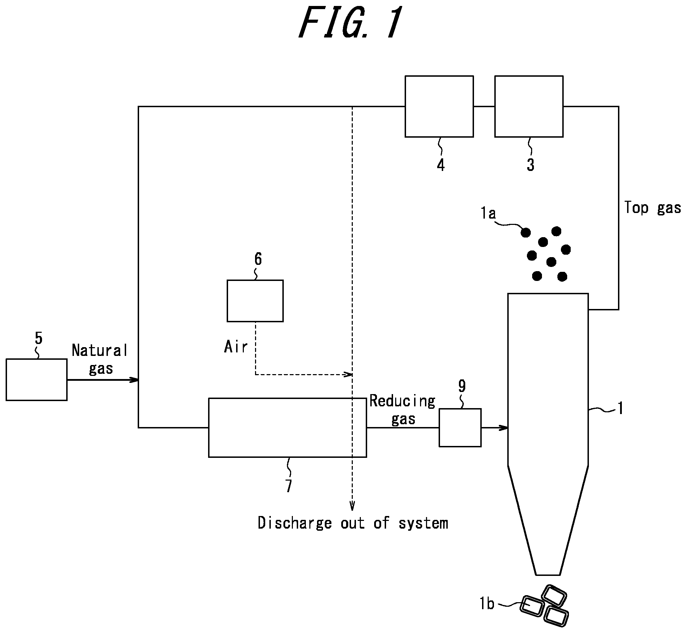

First, a conventional reduced iron production process (hereinafter also referred to as a conventional production process) is described. FIG. 1 is a schematic diagram illustrating an example of a conventional production process. In the drawing, reference sign 1 is a reduction furnace, 1a is iron oxide, 1b is reduced iron, 3 is a deduster, 4 is a dehydrator, 5 is a natural gas supply, 6 is an air supply, 7 is a reformer, and 9 is a reducing gas blowing device.

In the example of a conventional production process, iron oxide is charged from the top of the reduction furnace and gradually descends. There, iron oxide is reduced by blowing in high-temperature reducing gas from a central portion of the reduction furnace. The reduced iron is then discharged from the bottom of the reduction furnace. In this process, top gas containing mainly CO, $CO_2$, $H_2$, and $H_2O$ is discharged from the top of the reduction furnace. The top gas is de-dusted by the deduster, and a portion is subjected to moisture adjustment and fed to the reformer as feed gas. Gas containing hydrocarbons, for example, natural gas from a natural gas supply, is supplied to the reformer along with the top gas that has been subjected to moisture adjustment. The supplied gas is then heated in the reformer. A reforming reaction then occurs, producing high-temperature reducing gas containing mainly CO and $H_2$. This reducing gas is then blown into the reduction furnace. Further, the remaining portion of the top gas is dehydrated and used as fuel for heating in the combustion chamber of the reformer. After combustion as fuel for heating, the top gas still containing $CO_2$ is normally discharged out of the system. When reduced iron is produced using this example of a conventional production process, approximately a little more than 1 tonne of $CO_2$ is discharged out of the circulation system when 1 tonne of reduced iron is produced.

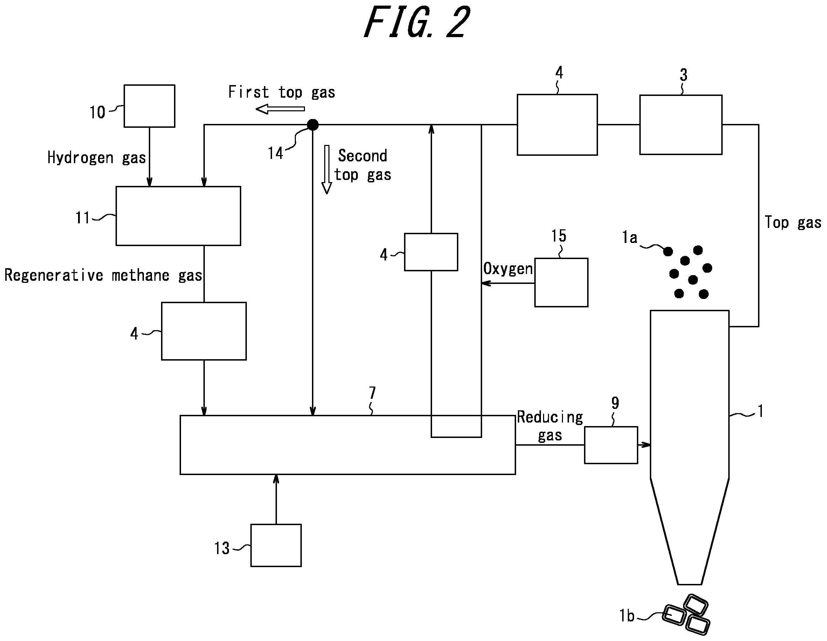

In contrast, in the method of producing reduced iron according to an embodiment of the present disclosure, as illustrated in FIG. 2 for example, the top gas discharged from the reduction furnace is distributed into first top gas and second top gas in a top gas distributor. Methane gas synthesized from the first top gas and $H_2$ gas (hereinafter also referred to as regenerative methane gas) is used instead of hydrocarbon gas such as natural gas supplied from outside, as in the conventional process illustrated in FIG. 1. In the drawing, reference sign 10 is a hydrogen supply, 11 is a methane synthesizer, 13 is a heat source, 14 is a top gas distributor, and 15 is an oxygen supply. Here, the regenerative methane gas produced in the methane synthesizer 11 is supplied to the reformer 7 to be used as feed gas for the reducing gas.

The following is a description of each process of the method of producing reduced iron according to an embodiment of the present disclosure. The charging process, blowing process, and reduction process can be carried out in accordance with conventional methods, for example, in the same manner as in the conventional process described above, and therefore description is omitted here.

• Distribution Process

In the distribution process, for example, the top gas discharged from the reduction furnace is distributed into the first top gas and the second top gas in the top gas distributor. Further, distribution of the top gas and a flow rate controller are not particularly limited and may follow a conventional method. For example, a mass flow controller or the like may be used.

In the distribution process, the top gas may be distributed in its original composition. Further, for example, certain gas types, such as $CO_2$ and $H_2$, may be separated and distributed as follows.

(a) The top gas is separated into separated carbon dioxide gas and first remaining gas ($CO_2$ separation), and the separated carbon dioxide gas is distributed as the first top gas and the first remaining gas as the second top gas.
(b) The top gas is separated into the separated carbon dioxide gas and the first remaining gas ($CO_2$ separation), and the separated carbon dioxide gas is distributed as the first top gas.

Further, the first remaining gas is separated into separated hydrogen gas and second remaining gas ($H_2$ separation). The separated hydrogen gas is then introduced (supplied) to the synthesis process and the second remaining gas is distributed as the second top gas.

(c) The top gas is separated into separated hydrogen gas and third remaining gas ($H_2$ separation). The separated hydrogen gas is then introduced (supplied) to the synthesis process, and the third remaining gas is separated into separated carbon dioxide gas and fourth remaining gas ($CO_2$ separation). The separated carbon dioxide gas is then distributed as the first top gas, and the fourth remaining gas is distributed as the second top gas.

The method of $CO_2$ separation is not particularly limited, and various methods may be used, examples including chemical absorption, physical absorption, adsorption separation, membrane separation, deep cold separation, oxygen combustion, chemical loop combustion, and the like. Among these, in particular, chemical absorption methods, of which an amine absorption method is representative, and adsorption separation methods, of which a PSA method is representative, have long been used in chemical plants, industrial $CO_2$ production, and the like. Further, these methods have a track record of recovering gas with a $CO_2$ concentration of 99 vol% or more. These methods are therefore preferred. The $CO_2$ concentration in the separated carbon dioxide gas is preferably 90 vol% or more. The $CO_2$ concentration in the separated carbon dioxide gas may be 100 vol%.

The method of $H_2$ separation is not particularly limited, and various methods may be used, examples including adsorption separation, membrane separation, and the like. The $H_2$ concentration in the separated hydrogen gas is preferably 90 vol% or more. The $H_2$ concentration in the separated hydrogen gas may be 100 vol%.

Further, the composition of the first remaining gas, the second remaining gas, and the fourth remaining gas distributed as the second top gas varies depending on the amount of $CO_2$ and $H_2$ separated, for example, CO: 5 vol% to 70 vol%, $CO_2$: 0 vol% to 25 vol%, $H_2$: 0 vol% to 75 vol%, $H_2O$: 0 vol% to 50 vol%, with the balance being: 0 vol% to 30 vol%.

When the top gas is distributed in the distribution process into the first top gas and the second top gas with the same composition, there may optionally be a separation process between the distribution process described above and the synthesis process described below to separate specific gas types such as $CO_2$ and $H_2$ from the first top gas and the second top gas.

For example, as described above under (a) and (b), the first top gas may be separated into separated carbon dioxide gas and first remaining gas ($CO_2$ separation), and the separated carbon dioxide gas may be used as the first top gas in the synthesis process. The first remaining gas described above may be merged directly into the second top gas. Further, the first remaining gas described above may be separated into separated hydrogen gas and the second remaining gas ($H_2$ separation), and then the separated hydrogen gas may be introduced into the synthesis process and the second remaining gas merged with the second top gas.

Further, as described under (c), the first top gas is separated into separated hydrogen gas and the third remaining gas. The separated hydrogen gas is then introduced into the synthesis process and the third remaining gas is separated into separated carbon dioxide gas and fourth remaining gas ($CO_2$ separation). The separated carbon dioxide gas may then be used as the first top gas in the synthesis process, and the fourth remaining gas may be merged with the second top gas.

Further, as illustrated in FIG. 2, from a mass balance perspective, some of the top gas may be used as fuel for heating, such as in a reformer, prior to introduction into the distribution process. For example, a portion of the top gas is burned in a combustion chamber of a heating apparatus using oxygen supplied from an oxygen supply, such as pure oxygen generated by a deep cold separation process driven by $CO_2$-free electrical power. The top gas after combustion is then dehydrated as required before being returned to the original line and introduced into the distribution process.

• Synthesis process

In the synthesis process, for example, regenerative methane gas is synthesized in a methane synthesizer from the first top gas distributed by the distribution process described above and hydrogen gas. $CH_4$ is synthesized from at least one of $CO_2$ or $CO$ in the first top gas and $H_2$ according to the following methanation reaction equations in Expressions (i) and (ii).

$$CO_2 + 4H_2 \rightarrow CH_4 + 2H_2O \ \Delta H = \text{-165 kJ/mol} \ \text{...} \qquad \text{(i)}$$

$$CO + 3H_2 \rightarrow CH_4 + H_2O \ \Delta H = \text{-206 kJ/mol} \ \text{...} \qquad \text{(ii)}$$

For example, the first top gas and hydrogen gas supplied from outside the circulation system described above are introduced into the methane synthesizer. $CH_4$ is then synthesized in the methane synthesizer by at least one of the reactions in Expressions (i) and (ii). Conditions for $CH_4$ synthesis are not particularly limited, and a conventional method may be followed.

When the separated carbon dioxide gas obtained in the distribution process or the separation process described above is used, the composition of the first top gas is the same as that of the separated carbon dioxide gas. Further, when the top gas is distributed in the distribution process with its original composition and no separation process is carried out, the composition of the first top gas is basically the same as the composition of the top gas introduced in the distribution process. In such a case, the composition of the first top gas is, for example, $CO$: 5 vol% to 50 vol%, $CO_2$: 5 vol% to 30 vol%, $H_2$: 5 vol% to 80 vol%, $H_2O$: 0 vol% to 35 vol%, with the balance being: 0 vol% to 20 vol%. The composition of the second top gas, described below, is similar.

In addition to the first top gas, any other gas containing at least one of $CO$ or $CO_2$ (hereinafter also referred to as "other gas") may be used in the synthesis process. Examples of the other gas include by-product gas of a steelmaking process, specifically blast furnace gas (BFG) and coke oven gas (COG). Further, the other gas may be introduced into the separation process described above along with the first top gas and separated into separated carbon dioxide gas and remaining gas before supplying the separated carbon dioxide gas to the synthesis process.

Further, the source of hydrogen gas used in the synthesis process is not particularly limited and the hydrogen gas may be supplied and produced by any method. Methods of producing hydrogen gas include, for example, synthesis by electrolysis of water and by decomposition reactions of ammonia, hydrocarbons, and organic hydrides. However, when hydrocarbons or organic hydrides are used as a raw material, $CO_2$ is emitted in the hydrogen synthesis process. Therefore, from the viewpoint of further decreasing $CO_2$ emissions, synthesis by at least one of the electrolysis of water or decomposition of ammonia is preferred. Further, when hydrogen gas is produced by electrolysis of water, green hydrogen produced using electrical power from green energy sources such as solar, wind, and geothermal power may be used to decrease $CO_2$ emissions to zero. The $H_2$ concentration of the hydrogen gas is not particularly limited. The $H_2$ concentration of the hydrogen gas is preferably 90 vol% or more. The $H_2$ concentration of the hydrogen gas is more preferably 95 vol% or more. The $H_2$ concentration of the hydrogen gas may be 100 vol%.

In the synthesis of $CH_4$, a typically used methanation catalyst may be used. Specifically, a transition metal catalyst such as Fe, Ni, Co, Ru, or the like may be used. Among these, Ni catalysts are the most active. Further, Ni catalysts also have high heat resistance and are usable up to about 500 °C. Therefore, Ni catalysts are particularly preferred. Further, iron ore may be used as a catalyst. Ores containing high crystallization water are particularly suitable for use as catalysts because the specific surface area increases when the water of crystallization is dehydrated.

The reactor of the methane synthesizer used in the synthesis process may be a fixed bed reactor, a fluidized bed reactor, an air flow bed reactor, or the like. Depending on the form of these reactors, the physical properties of the catalyst may be selected accordingly. Further, a heat exchanger may be disposed in the gas flow path downstream of the reactor to recover heat from the reaction heat (gas sensible heat) of the methanation reaction in each reactor. The recovered thermal energy may, for example, be used to heat the reduction furnace or the reformer.

Further, the methanation catalysts described above exhibit a stable high conversion rate during $CO_2$ methanation. However, catalyst poisoning occurs during CO methanation due to C precipitation caused by the formation of C intermediates. Examples of measures to prevent catalyst poisoning during CO methanation include the addition of $H_2O$ (water vapor). However, given the long-term use of the catalyst, a decrease in catalyst life is inevitable. Further, as indicated in Expressions (i) and (ii), the methanation reaction of CO has a larger negative reaction enthalpy and a generates more heat than the methanation reaction of $CO_2$. This causes a rapid increase in catalyst temperature during CO methanation. Further, the methanation reaction itself is an exothermic reaction, and therefore the conversion rate decreases with reaction time. Further, the increase in catalyst temperature may also accelerate catalyst deactivation. Therefore, the separated carbon dioxide gas obtained in the distribution process or the separation process is preferably used as the first top gas in the synthesis process.

When $H_2O$ produced as a byproduct of methane synthesis is introduced into the reformer, there may be an excess of $H_2O$ in the reforming process described below. Therefore, while considering the mass balance of the circulation system as a whole, it is preferable to dehydrate the regenerative methane gas using a dehydrator, as appropriate, prior to the reforming process described below.

Further, the $CH_4$ concentration of the regenerative methane gas is not particularly limited. In regenerative methane gas from which $H_2O$ is removed, that is, in regenerative methane gas after dehydration of the gas leaving the methane synthesizer by a dehydrator, the $CH_4$ concentration is preferably 80 vol% or more. The $CH_4$ concentration is more preferably 90 vol% or more. The $CH_4$ concentration in regenerative methane gas without $H_2O$ may be 100 vol%.

• Reforming process

In the reforming process, the regenerative methane gas and the second top gas are used as feed gas to obtain reducing gas from the feed gas. For example, the regenerative methane gas and the second top gas are introduced into the reformer, and the regenerative methane gas and the second top gas are heated in the reformer. Then, in the reformer, the reforming reactions of the following Expressions (iii) and (iv) produce the reducing gas containing CO and $H_2$. By supplying water vapor to the reformer, the reforming reaction represented in Expression (iv) proceeds.

$$CH_4 + CO_2 \rightarrow 2CO + 2H_2 \; \Delta H = 247 \text{ kJ/mol} ... \qquad \text{(iii)}$$

$$CH_4 + H_2O \rightarrow CO + 3H_2 \; \Delta H = 206 \text{ kJ/mol} ... \qquad \text{(iv)}$$

The feed gas may be heated in a reformer, for example. The heating temperature and method of heating the feed gas are not particularly limited and may be in accordance with a conventional method. For example, the heating temperature of the feed gas may be 300 °C to 700 °C. Further, the temperature of the reducing gas (blown into the reduction furnace) may be 750 °C to 1100 °C.

Further, the gas composition of the reducing gas is, for example, CO: 1 vol% to 60 vol%, $H_2$: 40 vol% to 99 vol%, with the balance being 0 vol% to 30 vol%.

As indicated by the positive enthalpies of formation in Expressions (iii) and (iv), both reactions in Expressions (iii) and (iv) are endothermic reactions. Accordingly, by using green energy, such as solar, wind, or geothermal energy, for example, as a heat source for the reformer, $CO_2$ emissions may in principle be decreased to zero.

The reducing gas is then introduced into the reduction furnace through a blowing process. For example, the reducing gas is introduced into the reduction furnace using a reducing gas blowing device. The iron oxide is then reduced by the reducing gas in the reduction furnace to obtain reduced iron. On the other hand, the reducing gas after being used for iron oxide reduction is discharged from the reduction furnace as the top gas.

Further, it is preferred that at least one of de-dusting or dehydration of the top gas is carried out prior to the distribution process. Any deduster may be used for de-dusting. Further, any dehydrator may be used for dehydration. The order of dedusting and dehydration is not particularly limited. In the example illustrated in FIG. 2, the top gas is dehydrated by the dehydrator after de-dusting by the deduster, and then the top gas is distributed into the first top gas and the second top gas.

• Control of amount of gas introduced into distribution process, synthesis process, and reforming process

In the method of producing reduced iron according to an embodiment of the present disclosure, it is important to control the amount of gas introduced (supplied) to the distribution process, the synthesis process, and the reforming process, according to the $CO_2$ conversion rate. This makes it possible to stabilize the composition of the reducing gas blown into the reduction furnace, particularly the ratio of the amount of $H_2$ to the amount of CO in the reducing gas, $H_2/CO$ (hereinafter also referred to as reducing gas $H_2/CO$). As a result, reduced iron can be produced with high operational stability without the need for extra energy input, that is, achieving both further energy savings and a decrease in $CO_2$ emissions.

Here, the $CO_2$ conversion rate indicates the ratio of $CO_2$ contained in the first top gas introduced into the synthesis process that is converted to $CH_4$ by the methanation reaction. For example, the $CO_2$ conversion rate may be calculated using the following expression.

$CO_2$ conversion ratio $\eta_m$ [-] = 1 - (amount of $CO_2$ contained in regenerative methane gas discharged after synthesis of $CH_4$ in synthesis process [$Nm^3/t$]) ÷ (amount of $CO_2$ contained in first top gas introduced into synthesis process [$Nm^3/t$])

The $CO_2$ conversion rate can be adjusted mainly by the reactor used in the synthesis process, for example, the type and amount of catalyst used in the reactor, the volume of the reactor, the temperature of the reactor, and the like. Further, when there are other gases introduced into the synthesis process besides the first top gas, such as the separated hydrogen gas, the amount of $CO_2$ contained in such gases is also included in (amount of $CO_2$ contained in first top gas introduced into synthesis process [$Nm^3/t$]).

Further, $Nm^3/t$ and kg/t are the basic units per tonne of reduced iron (DRI) produced.

For example, it is suitable to control the amount of gas introduced into the distribution process, the synthesis process, and the reforming process to satisfy the following Expressions (1) and (2).

[Math 3]

$$1 - \frac{V_{CO2}}{W_{CO2}} \leq \eta_m \qquad \ldots(1)$$

[Math 4]

$$1 - \frac{V_{H2}}{W_{H2}+E_{H2}} \leq \eta_m \leq 1 + \frac{W_{H2}-V_{H2}}{E_{H2}} \qquad \ldots(2)$$

Here,

$W_{CO2}$ is an amount of $CO_2$, in $Nm^3/t$, contained in the top gas introduced into the distribution process,
$W_{H2}$ is an amount of $H_2$, in $Nm^3/t$, contained in the top gas introduced into the distribution process,
$E_{H2}$ is an amount of $H_2$, in $Nm^3/t$, contained in the hydrogen gas introduced into the synthesis process,
$V_{CO2}$ is an amount of $CO_2$, in $Nm^3/t$, contained in the gas introduced into the reforming process, and
$V_{H2}$ is an amount of $H_2$, in $Nm^3/t$, contained in the gas introduced into the reforming process.

The gases introduced into the reforming process are basically the regenerative methane gas and the second top gas. The hydrogen gas introduced into the synthesis process is hydrogen gas introduced from outside the circulation system, and does not include the separated hydrogen gas described above or the $H_2$ in the first top gas.

The left side of Expression (1) above can be rewritten as $(W_{CO2} - V_{CO2})/W_{CO2}$, which represents the ratio of $CO_2$ that is converted to $CH_4$ out of the $CO_2$ introduced into the distribution process. When Expression (1) is not satisfied, the amount of $CO_2$ converted to $CH_4$ in the synthesis process is not sufficient, resulting in a $CO_2$ surplus in the circulation system. As a result, stable operation under a healthy mass balance may require the discharge of $CO_2$ out of the circulation system.

Further, the left side of Expression (2) can be rewritten as $(W_{H2} + E_{H2} - V_{H2}) / (W_{H2} + E_{H2})$, which represents the

proportion of $H_2$ that is converted to $CH_4$ out of the total $H_2$ introduced during processing from the distribution process to the reforming process. When the $CO_2$ conversion rate is less than the left-side value of Expression (2), the reaction efficiency in the synthesis process is not sufficient, resulting in a $H_2$ surplus in the circulation system. As a result, stable operation under a healthy mass balance may become impossible.

The right side of Expression (2) can be rewritten as $(W_{H2} + E_{H2} - V_{H2}) / E_{H2}$, which represents the proportion of the amount of $H_2$ converted to $CH_4$ relative to the amount of $H_2$ introduced from outside the circulation system in the synthesis process. When the $CO_2$ conversion ratio $\eta$ exceeds the right-side value of Expression (2), excessive $H_2$ is consumed in the synthesis process, which is undesirable from the viewpoint of energy savings.

In view of the above, the amount of gas introduced into the distribution process, the synthesis process, and the reforming process is preferably controlled so that Expressions (1) and (2) are both satisfied.

The left side of Expression (1) is more preferably $1 - V_{CO2}/W_{CO2} + 0.015$.

The left side of Expression (2) is more preferably $1 - V_{H2} / (W_{H2} + E_{H2}) + 0.015$.

The right side of Expression (2) is more preferably $1 + (W_{H2} - V_{H2}) / E_{H2} - 0.015$.

The amount of gas introduced into the distribution process, the synthesis process, and the reforming process may be constant or may be changed at any time, as long as Expressions (1) and (2) are both satisfied. The amount of gas to be introduced into the distribution process, the synthesis process, and the reforming process at the time of facility startup may be determined, for example, from past operating history, and the amount of gas to be introduced into the distribution process, the synthesis process, and the reforming process may be changed and controlled as appropriate according to subsequent operating conditions.

Further, the iron oxide raw material used in the method of producing reduced iron according to an embodiment of the present disclosure is, for example, iron ore. Examples include lumped iron ore (lump ore), pellets (powdered iron ore hardened into a spherical shape), and the like. The grade of iron ore used as iron oxide raw material, that is, the iron content, is not particularly limited. From the perspective of reduction in a shaft furnace, the iron content is typically preferably 65 mass% or more.

In addition, the method of producing reduced iron according to an embodiment of the present disclosure describes, among other things, the use of a shaft furnace as a direct reduction iron-making process. However, the type of reduction furnace is not limited to this, and may be a fluidized bed, a rotary kiln, a rotary hearth furnace (RHF), or the like. Shaft furnaces are preferred as reduction furnaces because of their high production efficiency, ratio of utilization and operational stability. Further, the majority of direct reduction furnaces in operation worldwide are Midrex® (Midrex is a registered trademark in Japan, other countries, or both) and Hyl® (Hyl is a registered trademark in Japan, other countries, or both), which are shaft furnace systems.

EXAMPLES

Examples of the present disclosure are described below.

In the circulation system illustrated in FIG. 2, reduced iron was produced according to the conditions listed in Table 1. Under all conditions, the operation period was 28 days. In Table 1, the operating parameters are listed in terms of basic unit per tonne of reduced iron produced. For example, when 1300 kg of iron oxide pellets are used to produce 1 tonne of reduced iron, the amount of iron oxide pellets used is expressed as 1300 kg/t. When 3000 t/d of reduced iron is to be produced, this amount can be multiplied by 3000 to obtain the daily parameters.

Here, under all conditions, iron oxide pellets as raw material were charged into the reduction furnace at 1394 kg/t in a charging process. In the blowing process, reducing gas heated to 980 °C was blown in from a middle portion of the reduction furnace to reduce the iron oxide pellets to obtain reduced iron. The top gas discharged from the reduction furnace was then dedusted, then dehydrated as appropriate to balance the mass balance. From the viewpoint of mass balance, a portion of the top gas was used as fuel for heating, as appropriate. The top gas diverted as fuel for heating was burned in the combustion chamber of the reformer using oxygen generated by a deep-cooling separation process driven by $CO_2$-free electrical power. All the exhaust gas from the combustion chamber of the reformer was then recovered, dehydrated, and then re-mixed with the top gas. The top gas after the re-mixing was then introduced into the distribution process and distributed into the first top gas and the second top gas. In the distribution process, the top gas introduced into the top gas distributor was distributed as the first top gas by obtaining separated carbon dioxide gas according to any of the above configurations (a) to (c), as appropriate, so that the mass balance is balanced. Separated hydrogen gas was also introduced into the synthesis process as the first top gas, along with the separated carbon dioxide gas. Further, the remaining gas (the first remaining gas, the second remaining gas, or the fourth remaining gas) was distributed as the second top gas. The first top gas (including some separated hydrogen gas) and hydrogen gas from outside the circulation system were then introduced into the methane synthesizer, and regenerative methane gas was synthesized in the reactor of the methane synthesizer. After dehydration of the synthesized regenerative methane gas, regenerative methane gas and the second top gas were introduced into the reformer as feed gas, and from the feed gas, reducing gas was obtained. The total amount of introduced gas for the reforming process in Table 1 is the sum of the regenerative methane gas and the second top gas. Conditions other than those described above and in Table 1 were in accordance with a conventional method.

[Table 1]

Table 1

| Parameters | | Unit | Example 1 | Example 2 | Example 3 | Example 4 | Comparative Example 1 | Comparative Example 2 | Comparative Example 3 | Comparative Example 4 |
|---|---|---|---|---|---|---|---|---|---|---|---|
| Reducing gas $H_2$/CO | | - | 0.82 | 1.49 | 1.86 | 2.33 | 0.82 | 1.49 | 2.33 | 3.00 |
| Distribution process | Introduced top gas amount | $Nm^3$/t | 1492 | 1679 | 1932 | 2107 | 1492 | 1679 | 2107 | 2339 |
| | $W_{CO2}$ | $Nm^3$/t | 267 | 628 | 170 | 147 | 267 | 628 | 147 | 122 |
| | $W_{H2}$ | $Nm^3$/t | 519 | 495 | 1087 | 1279 | 519 | 495 | 1279 | 1571 |
| Synthesis process | $E_{H2}$ | $Nm^3$/t | 882 | 940 | 1122 | 1196 | 882 | 940 | 1196 | 1328 |
| Reforming process | Total amount of introduced gas | $Nm^3$/t | 1492 | 1655 | 1973 | 2121 | 1492 | 1655 | 2121 | 2412 |
| | $V_{CO2}$ | $Nm^3$/t | 132 | 579 | 35 | 12 | 132 | 579 | 12 | 0 |
| | $V_{H2}$ | $Nm^3$/t | 519 | 248 | 1087 | 1279 | 519 | 248 | 1279 | 1627 |
| $CO_2$ conversion rate $\eta_m$ | | - | 0.65 | 0.85 | 0.85 | 0.95 | 0.55 | 0.75 | 0.85 | 0.97 |
| Control of gas amount by $CO_2$ conversion rate | | - | Yes | Yes | Yes | Yes | No | No | No | No |
| Expression (1) left-side value | | - | 0.51 | 0.08 | 0.80 | 0.92 | 0.51 | 0.08 | 0.92 | 1.00 |
| Expression (2) left-side value | | - | 0.63 | 0.83 | 0.51 | 0.48 | 0.63 | 0.83 | 0.48 | 0.44 |
| Expression (2) right-side value | | - | 1.00 | 1.26 | 1.00 | 1.00 | 1.00 | 1.26 | 1.00 | 0.96 |
| Are both Expressions (1) and (2) satisfied? | | - | Yes | Yes | Yes | Yes | No | No | No | No |

For all of the Examples, the circulation system illustrated in FIG. 2, which is extremely advantageous in realizing energy savings, that is, a system in which top gas is circulated and reused, was able to operate stably under a healthy mass balance for the entire 28-day operation period. Further, $CO_2$ emissions from the circulation system could be decreased to zero.

On the other hand, for all of the Comparative Examples where the gas volume was not controlled according to the $CO_2$ conversion ratio in the synthesis process, the reaction in the reduction furnace became unstable during the operation period because the composition of the reducing gas could no longer be maintained within a certain range, and the operation had to be suspended.

REFERENCE SIGNS LIST

| | |
|---|---|
| 1 | reduction furnace |
| 1a | iron oxide |
| 1b | reduced iron |
| 3 | deduster |
| 4 | dehydrator |
| 5 | natural gas supply |
| 6 | air supply |
| 7 | reformer |
| 9 | reducing gas blowing device |
| 10 | hydrogen supply |
| 11 | methane synthesizer |
| 13 | heat source |
| 14 | top gas distributor |
| 15 | hydrogen supply |

**Claims**

1. A method of producing reduced iron, the method comprising:

   a charging process of charging iron oxide into a reduction furnace;
   a blowing process of blowing reducing gas into the reduction furnace;
   a reduction process, in the reduction furnace, of reducing the iron oxide by the reducing gas to obtain reduced iron;
   a distribution process of distributing top gas discharged from the reduction furnace into a first top gas and a second top gas;
   a synthesis process of synthesizing regenerative methane gas using the first top gas and hydrogen gas; and
   a reforming process of using the regenerative methane gas and the second top gas as feed gas to obtain the reducing gas from the feed gas, wherein
   an amount of gas introduced into the distribution process, the synthesis process, and the reforming process is controlled according to a $CO_2$ conversion rate $\eta_m$ from a methanation reaction in the synthesis process.

2. The method of producing reduced iron according to claim 1, wherein the amount of gas introduced into the distribution process, the synthesis process, and the reforming process is controlled so as to satisfy the following Expressions (1) and (2),
[Math 1]

$$1 - \frac{V_{CO2}}{W_{CO2}} \leq \eta_m$$

$$\dots(1)$$

[Math 2]

$$1 - \frac{V_{H2}}{W_{H2} + E_{H2}} \leq \eta_m \leq 1 + \frac{W_{H2} - V_{H2}}{E_{H2}} \qquad \ldots(2)$$

where

$W_{CO2}$ is an amount of $CO_2$, in $Nm^3/t$, contained in the top gas introduced into the distribution process,
$W_{H2}$ is an amount of $H_2$, in $Nm^3/t$, contained in the top gas introduced into the distribution process,
$E_{H2}$ is an amount of $H_2$, in $Nm^3/t$, contained in the hydrogen gas introduced into the synthesis process,
$V_{CO2}$ is an amount of $CO_2$, in $Nm^3/t$, contained in the gas introduced into the reforming process, and
$V_{H2}$ is an amount of $H_2$, in $Nm^3/t$, contained in the gas introduced into the reforming process.

3. The method of producing reduced iron according to claim 1 or 2, wherein, in the distribution process, the top gas is separated into separated carbon dioxide gas and first remaining gas,

the separated carbon dioxide gas is distributed as the first top gas, and
the first remaining gas is distributed as the second top gas.

4. The method of producing reduced iron according to claim 1 or 2, wherein, in the distribution process, the top gas is separated into separated carbon dioxide gas and first remaining gas,

the separated carbon dioxide gas is distributed as the first top gas,
the first remaining gas is separated into separated hydrogen gas and second remaining gas,
the separated hydrogen gas is introduced into the synthesis process, and
the second remaining gas is distributed as the second top gas.

5. The method of producing reduced iron according to claim 1 or 2, wherein, in the distribution process, the top gas is separated into separated hydrogen gas and third remaining gas,

the separated hydrogen gas is introduced into the synthesis process,
the third remaining gas is separated into separated carbon dioxide gas and fourth remaining gas, and
the separated carbon dioxide gas is distributed as the first top gas and the fourth remaining gas is distributed as the second top gas.

EP 4 621 076 A1

# FIG. 1

FIG. 2

## INTERNATIONAL SEARCH REPORT

| International application No. |
| --- |
| **PCT/JP2023/045555** |

**A. CLASSIFICATION OF SUBJECT MATTER**

*C21B 5/06*(2006.01)i; *C07C 1/04*(2006.01)i; *C07C 1/12*(2006.01)i; *C10K 3/06*(2006.01)i; *C21B 5/00*(2006.01)i; *C21B 13/02*(2006.01)i; *F27D 17/00*(2006.01)i
FI: C21B5/06; C07C1/04; C07C1/12; C10K3/06; C21B5/00 321; C21B13/02; F27D17/00 104G

According to International Patent Classification (IPC) or to both national classification and IPC

**B. FIELDS SEARCHED**

Minimum documentation searched (classification system followed by classification symbols)

C21B5/06; C07C1/04; C07C1/12; C10K3/06; C21B5/00; C21B13/02; F27D17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2014-028984 A (JFE STEEL CORPORATION) 13 February 2014 (2014-02-13) paragraphs [0001]-[0033], fig. 1 | 1, 3-5 |
| A | | 2 |

☐ Further documents are listed in the continuation of Box C.  ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **13 March 2024** | **26 March 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2023/045555**

| Patent document cited in search report | Publication date (day/month/year) | Patent family member(s) | Publication date (day/month/year) |
|---|---|---|---|
| JP 2014-028984 A | 13 February 2014 | (Family: none) | |

Form PCT/ISA/210 (patent family annex) (July 2022)

**REFERENCES CITED IN THE DESCRIPTION**

**Patent documents cited in the description**

- JP 2017088912 A

- JP 6190522 B